# EUROPEAN PATENT APPLICATION

(11) **EP 2 140 885 A1**
(43) Date of publication of application: **06.01.2010**
(21) Application number: 09163232.3
(22) Date of filing: 19.06.2009
(51) Int. Cl.: A61L 2/10, A61L 2/26, A46B 17/06

(54) **Toothbrush sterilizer**

(30) Priority: 27.06.2008 JP 2008169145
(71) Applicant: Panasonic Electric Works Co., Ltd., Kadoma 571-8686 Osaka (JP)
(72) Inventor: Kitagawa, Tadanobu, Kadoma 571-8686 Osaka (JP); Kitamura, Yoshihiro, Kadoma 571-8686 Osaka (JP)
(74) Representative: Skuhra, Udo

(57) **Abstract**

A toothbrush sterilizer (1) includes a housing (10) having an opening (K). A door (12) opens and closes the opening (K). A sterilization element (17) sterilizes a toothbrush (H).
A toothbrush holding portion (30) holds the toothbrush (H). The sterilization element (17) and the toothbrush holding portion (30) are accommodated in the housing (10). The toothbrush holding portion (30) moves out of the housing (10) through the opening (K) in cooperation with an opening operation of the door (12). Further, the toothbrush holding portion (30) moves into the housing (10) through the opening (K) in cooperation with a closing operation of the door (12).

## Description

This application claims the benefit of Japanese Patent Application No. 2008-169415, filed on June 27, 2008, the entire contents of which are incorporated herein by reference.

The present invention relates to a toothbrush sterilizer that holds and sterilizes a toothbrush.

Various types of toothbrush sterilizers for sterilizing toothbrushes and holding toothbrushes in a hygienic state have been proposed. A toothbrush sterilizer includes a main body, which accommodates a toothbrush, and a sterilization lamp, which is arranged in the main body to irradiate the bristle portion of the toothbrush with ultraviolet rays (see Japanese Laid-Open Patent No. 2000-325442). This toothbrush sterilizer includes a door, which is attached to the main body, and a tooth brush holding portion (toothbrush supporting board), which has a holding receptacle for holding the toothbrush. When the door is closed, the toothbrush holding portion is accommodated in the main body. After opening the door, the toothbrush holding portion is manually drawn out of the main body.

However, this toothbrush sterilizer is inconvenient because at least two operations are required to use the toothbrush held in the toothbrush sterilizer, the operation for opening the door and the operation for drawing out the toothbrush holding portion. Furthermore, since the two operations are necessary to remove the toothbrush from the toothbrush sterilizer after a sterilization process, the user may inadvertently touch the bristle portion of the toothbrush. This may re-contaminate the bristle portion with bacteria.

It is an object of the present invention to provide a toothbrush sterilizer that is convenient and improves hygienics.

One aspect of the present invention is a toothbrush sterilizer for sterilizing a toothbrush.
The toothbrush sterilizer includes a housing having an opening, a door which opens and closes the opening, a sterilization element, and a toothbrush holding portion which holds the toothbrush. The sterilization element and the toothbrush holding portion are accommodated in the housing, and the sterilization element sterilizes the toothbrush. The toothbrush holding portion moves out of the housing through the opening in cooperation with an opening operation of the door, and the toothbrush holding portion moves into the housing through the opening in cooperation with a closing operation of the door.

Other aspects and advantages of the present invention will become apparent from the following description, taken in conjunction with the accompanying drawings, illustrating by way of example the principles of the invention.

The invention, together with objects and advantages thereof, may best be understood by reference to the following description of the presently preferred embodiments together with the accompanying drawings in which:
Fig. 1 is a perspective view showing a toothbrush sterilizer in a state in which a door is open;
Fig. 2 is a perspective view showing the toothbrush sterilizer of Fig. 1 from a diagonally lower position;
Fig. 3 is a plan view showing the bottom surface of a toothbrush holding tray;
Fig. 4 is a front view showing the toothbrush sterilizer in a state in which the door is closed;
Fig. 5 is a cross-sectional view of the toothbrush sterilizer of Fig. 4;
Figs. 6(a) to 6(e) are cross-sectional views illustrating the cooperation of the opening operation of the door and the drawing operation of the toothbrush holding tray;
Figs. 7(a) to 7(f) are cross-sectional views illustrating the cooperation of the closing operation of the door and the drawing operation of the toothbrush holding tray;
Figs. 8(a) and (b) and 9 are perspective views illustrating the detachment operation of the toothbrush holding tray; and
Figs. 10(a) and (b) are plan views showing a modification of a toothbrush sterilizer having a door located at a different position.

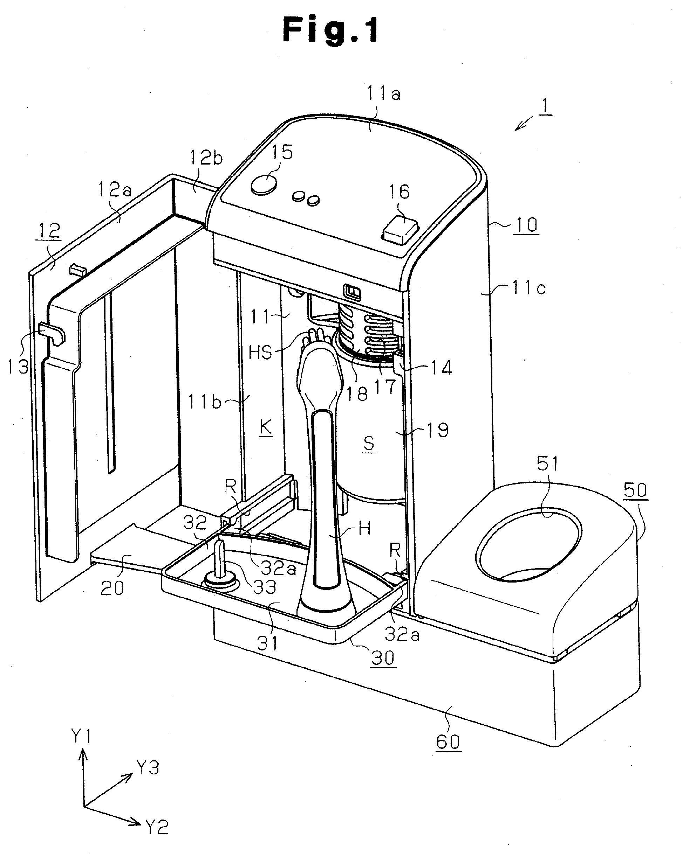

A preferred and illustrated embodiment of a toothbrush sterilizer according to the present invention will now be discussed. In this embodiment, the toothbrush sterilizer may be arranged and used on a washstand. The toothbrush sterilizer particularly sterilizes the bristle portion of a toothbrush. The arrows Y1, Y2, and Y3 in Fig. 1 respectively indicate the upward direction, the sideward direction, and the backward direction (depthwise direction).

As shown in Figs. 1, 2, and 4, the toothbrush sterilizer 1 includes a block-shaped base 60, a housing 10 arranged on the base 60, and an electric toothbrush holder 50 for holding an electric toothbrush in an upright state.

The housing 10 has a front side that includes an opening K. The opening K is defined in the housing 10 in front of a rear panel 11 between the front edges of a top panel 11a, a left panel 11b, and a right panel 11c. In the illustrated example, the dimension of the left panel 11b in the depthwise direction Y3 is about half the dimension of the right panel 11c in the depthwise direction Y3.

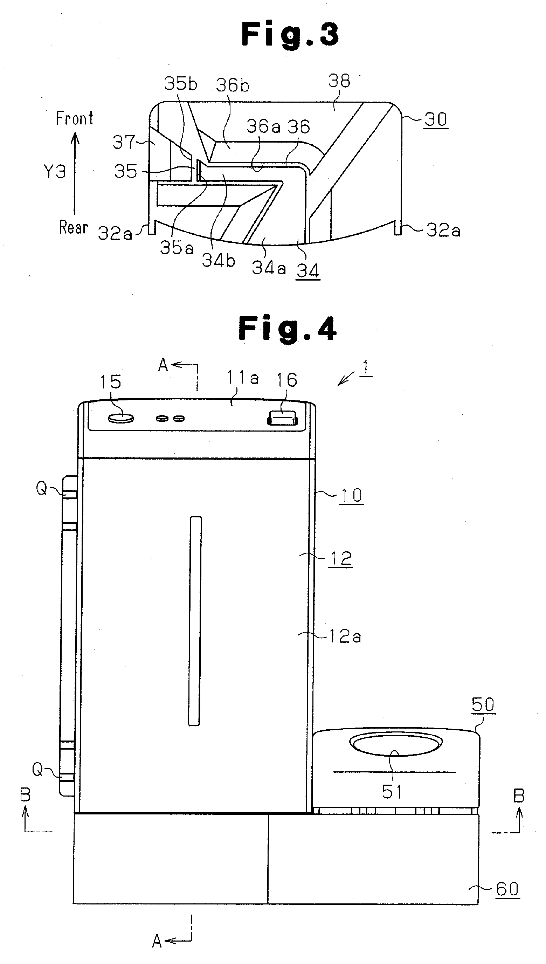

A door 12 for opening and closing the opening K is attached to the housing 10. In the illustrated example, the door 12 is attached to an outer side surface of the left panel 11b near the front edge. The door 12 is L-shaped and includes a front plate 12a, which corresponds to the opening K, and a side plate 12b, which is fixed to the front plate 12a at a generally right angle. The door 12 is attached to one end of the left panel 11b by support shafts Q (see Fig. 4) or rotation shafts. In the illustrated example, the support shafts Q are arranged on the door 12, and the left panel 11b receives the support shaft Q. In another example, the support shafts Q are arranged on the left panel 11b, and the door 12 receives the support shafts Q.

When the door 12 closes the opening K, the side plate 12b is flush with the left panel 11b, and the opening K is closed by the front plate 12a. The dimension of the front plate 12a in the sideward direction Y2 when the door 12 is closed is substantially the same as the dimension of the rear panel 11 in the sideward direction Y2. The dimension of the front plate 12a in the upward direction Y1 when the door 12 is closed is substantially the same as the dimension of the left panel 11b in the upward direction Y1. The total dimension of the left panel 11b and the side plate 12b in the depthwise direction Y3 when the door 12 is closed is substantially the same as the dimension of the right panel 11c in the depthwise direction Y3. The dimension of the side plate 12b in the depthwise direction Y3 when the door 12 is closed is substantially the same as the dimension of the left panel 11b in the depthwise direction Y3. That is, the support shafts Q are arranged at substantially halfway positions of the toothbrush sterilizer 1 in the depthwise direction Y3.

An engagement piece 13, which is substantially L-shaped, is formed on the upper inner part of the front plate 12a to engage the housing 10 when the door 12 is closed. An engagement groove 14 is formed on the right panel 11c of the housing 10 to engage the engagement piece 13. Engagement of the engagement piece 13 and the engagement groove 14 holds the door 12 in a state hooked to the housing 10, that is, in a state closing the opening K.

A power switch 15, which is pushed when switching ON and OFF the power of the toothbrush sterilizer 1, and a door open switch 16, which opens the door 12, are arranged on the upper surface of the top panel 11a.

The interior of the housing 10 serves as an accommodation chamber S. A sterilization element is arranged in the accommodation chamber S near the rear panel 11 to sterilize a bristle portion HS of the toothbrush H so that the toothbrush H becomes free from bacteria. The sterilization element is a UV lamp 17 or the like. The UV lamp 17 is surrounded by a first cover 18 including a plurality of holes. The first cover 18 prevents a user's fingers and the bristle portion HS of the toothbrush H from directly contacting the UV lamp 17. Furthermore, the UV lamp 17 has a lower part surrounded by a substantially cylindrical second cover 19.

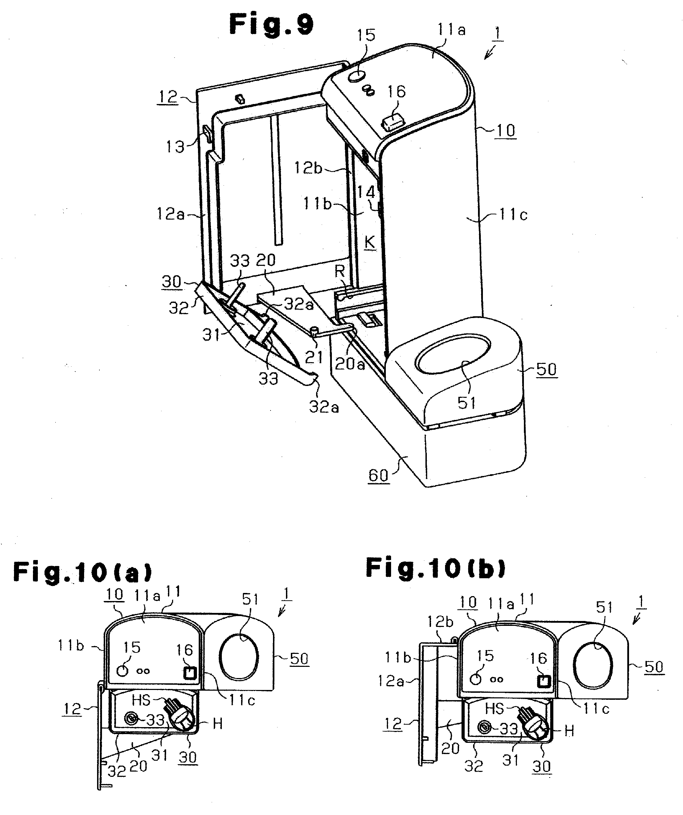

A rail R, which is formed by a constant groove and extends in the depthwise direction Y3 of the housing 10, is arranged on the lower inner surface of each of the left panel 11b and the right panel 11c. A small protrusion Rp projects downward from the front end of each rail R (see Fig. 9).

In the accommodation chamber S, a toothbrush holding tray 30, which serves as a toothbrush holding portion, is arranged in front of the UV lamp 17. The toothbrush holding tray 30 is guided by the rails R and movable in the depthwise direction Y3. The toothbrush holding tray 30 has a substantially rectangular shape. The toothbrush holding tray 30 includes a holder surface (top surface) 31 and a peripheral wall 32. The peripheral wall 32 has a height (dimension in the upward direction Y1) that is slightly smaller than the groove of the rail R. The peripheral wall 32 is guided by the grooves of the rails R. The toothbrush holding tray 30 moves in the depthwise direction Y3 along the rails R.

The toothbrush holding tray 30 includes extensions 32a extending toward the rear from the peripheral wall 32 (see Figs. 3 and 9). The extensions 32a are gradually curved in an upward direction so as to engage the corresponding protrusions Rp of the rail R. This determines the outermost drawn position of the toothbrush holding tray 30.

The dimension of the toothbrush holding tray 30 in the sideward direction Y2 is shorter than the dimension of the accommodation chamber S in the sideward direction Y2. The dimension of the toothbrush holding tray 30 in the depthwise direction Y3 is about half the dimension of the accommodation chamber S in the depthwise direction Y3. The housing chamber S houses both the UV lamp 17 and the toothbrush holding tray 30. The dimension of the accommodation chamber S in the upward direction Y1 is slightly greater than the length of the toothbrush H.

The holder surface 31 of the toothbrush holding tray 30 includes a plurality of (two in this embodiment) projection pieces 33, which are spaced apart from each other by a predetermined distance and project upward. The toothbrush H is attached to a main body (also referred to as a grip) of an electric toothbrush. The toothbrush H includes an attachment hole (not shown) so that it can be attached to the electric toothbrush main body. The attachment hole extends from a basal portion of the toothbrush H towards the bristle portion HS of the toothbrush H. Each projection piece 33 of the toothbrush holding tray 30 is shaped so that it can be fitted into the attachment hole of the toothbrush H. The toothbrush H is held in an upright state by fitting the projection piece 33 into its attachment hole. The distance between the projection pieces 33 is determined so that toothbrushes H held in an upright state do not contact each other. Each projection piece 33 extends in a direction (angle) determined so that the bristle portion HS of the corresponding toothbrush H faces the UV lamp 17.

The electric toothbrush holder 50 includes a charging socket 51 for receiving an electric toothbrush. The charging socket 51 holds and charges an electric toothbrush in an upright state.

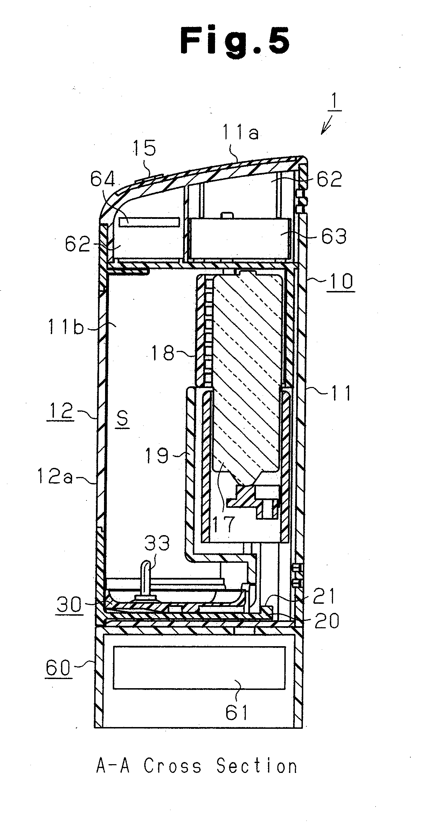

Referring to Fig. 5, the base 60 accommodates a lower control circuit 61, which controls the charging performed by the charging socket 51 and the irradiation intensity of the UV lamp 17. An upper accommodation chamber 62 is defined above the accommodation chamber S. The upper accommodation chamber 62 accommodates a fan 63, which dries the interior of the accommodation chamber S. The upper accommodation chamber 62 also accommodates an upper control circuit 64, which activates the toothbrush sterilizer 1 when the fan 63 is operated and the power switch 15 is pushed. In this embodiment, the lower control circuit 61 may also include timer function for automatically stopping the irradiation of ultraviolet rays from the UV lamp 17 after a predetermined time elapses.

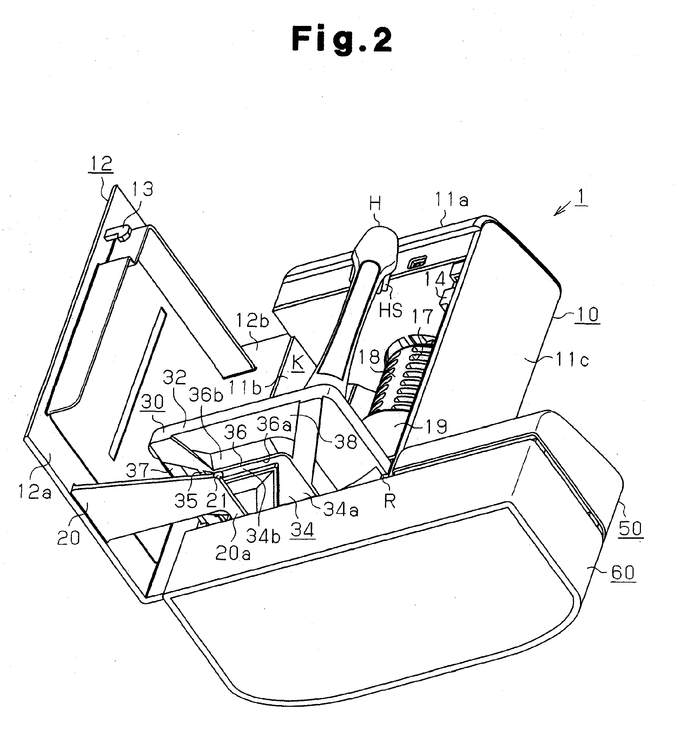

The toothbrush sterilizer 1 is formed so that the toothbrush holding tray 30 moves in cooperation with the opening operation and closing operation of the door 12. This cooperative operation will now be discussed with reference to Figs. 2 and 3. In the following description, the front side of the toothbrush sterilizer 1 may be referred to as the opening side, and the rear side of the toothbrush sterilizer 1 may be referred to as a farther side of the accommodation chamber S.

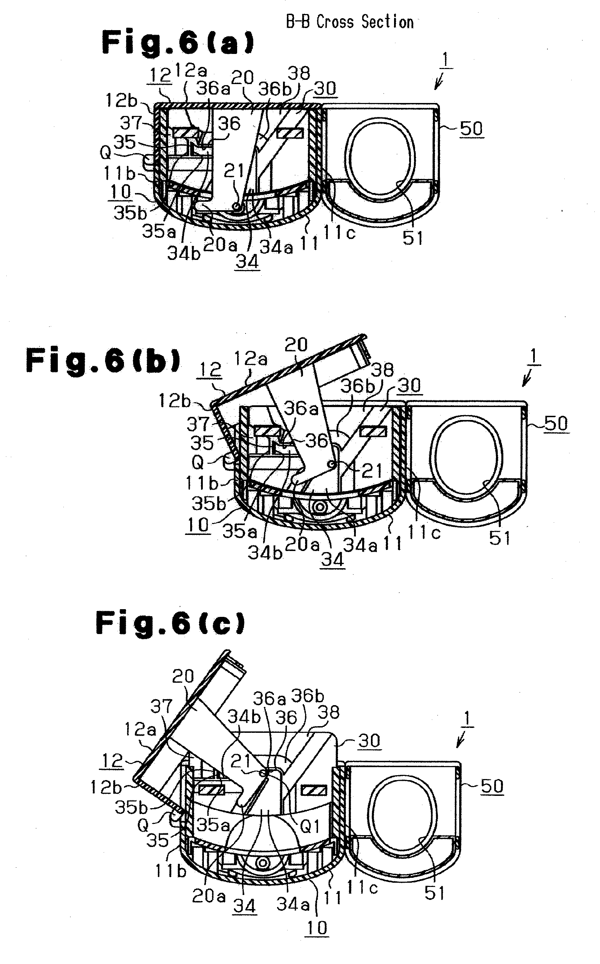

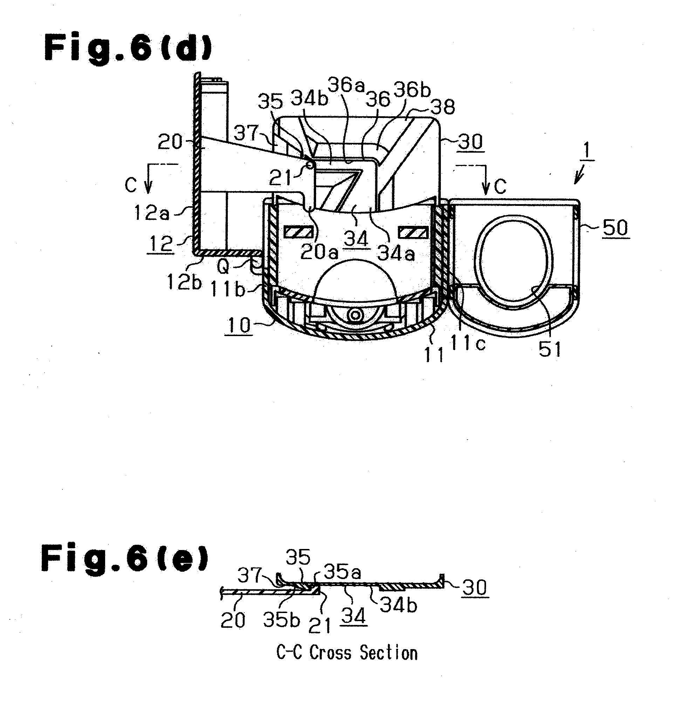

A connecting plate 20, which serves as a connecting member, extends from the lower inner surface of the front plate 12a of the door 12 and connects to the lower surface of the toothbrush holding tray 30. The connecting plate 20 extends horizontally and perpendicular to the inner surface of the front plate 12a. The connecting plate 20 is substantially L-shaped and flexible. The connecting plate 20 has a longitudinal dimension, or length, determined so as to enable movement of the toothbrush holding tray 30, as will be described in detail later. The connecting plate 20 has a basal portion, which is fixed to the door 12, and a distal portion, which includes a cylindrical protrusion 21 projecting upward and a projection 20a projecting sideward in a direction that is substantially parallel to the surface of the front plate 12a (see Figs. 6, 7, and 9). In the example of Fig. 9, the protrusion 21 and the projection 20a are formed on the connecting plate 20 at opposite sides of the distal portion. The distal portion of the connecting plate 20 may be referred to as a free end.

Fig. 3 shows the bottom surface of the toothbrush holding tray 30 opposite to the holder surface 31. The bottom surface is an example of a guide surface. A guide groove 34a has an open end located at substantially the middle of the rear side of the toothbrush holding tray 30. The guide groove 34a extends toward the front from the rear side of the lower surface of the toothbrush holding tray 30. The guide groove 34a has a width that gradually narrows towards the front. The guide groove 34a has a front end that is in communication with a guide groove 34b, which extends in a direction (Y2) orthogonal to the guide groove 34a. The guide groove 34b extends towards substantially the middle of the left side of the toothbrush holding tray 30 and has a terminal end defined by a first restriction wall 35. The guide groove 34a and the guide groove 34b define an L-shaped first guide groove 34. Most of the guide groove 34b has a substantially uniform width. However, the terminal end portion of the guide groove 34b is formed to widen towards the front.

The width of the guide groove 34a is much greater than the diameter of the protrusion 21 on the connecting plate 20. The width of the guide groove 34b is slightly larger than the diameter of the protrusion 21. The protrusion 21 of the connecting plate 20 moves along the first guide groove 34. In this manner, the connecting plate 20 connects the door 12 and the toothbrush holding tray 30. The guide groove 34a is formed so that the protrusion 21 does not become caught therein. The guide groove 34b is formed so as to provide a certain amount for the protrusion 21 while substantially restricting movement of the protrusion 21 to the sideward direction Y2.

The toothbrush holding tray 30 includes a second guide groove 37, which has an open end located at substantially the middle of the left side of the toothbrush holding tray 30 and a closed terminal end defined by the first restriction wall 35. The second guide groove 37 has a width that gradually narrows from the open end towards the terminal end. The second guide groove 37 is trapezoidal. The width of the second guide groove 37 is much greater than the diameter of the protrusion 21 on the connecting plate 20. The protrusion 21 moves along the second guide groove 37. The second guide groove 37 is formed so that the protrusion 21 does not become caught therein.

The first restriction wall 35 includes a wall surface 35a (also referred to as a steep inclined surface), which has a steep gradient and defines the terminal end portion of the guide groove 34b, and a wall surface 35b (also referred to as a gradual inclined surface), which has gradual gradient and defines the terminal end portion of the second guide groove 37. In other words, the inclination angle of the wall surface 35b is smaller than the inclination angle of the wall surface 35a. Thus, when the protrusion 21 is guided from the guide groove 34b toward the first restriction wall 35, the protrusion 21 becomes difficult to move from the guide groove 34b to the second guide groove 37 since the wall surface 35a has a steep gradient. When the protrusion 21 is guided from the second guide groove 37 toward the first restriction wall 35, the protrusion 21 easily moves from the second guide groove 37 to the guide groove 34b since the wall surface 35b has a gradual gradient.

The toothbrush holding tray 30 further includes a third guide groove 38 having a front open end. The third guide groove 38 has a closed terminal end defined by a second restriction wall 36. The third guide groove 38 has a width that gradually narrows from the open end towards the terminal end. The third guide groove 38 is trapezoidal. The third guide groove 38 is larger in area than the second guide groove 37. The width of the third guide groove 38 is much greater than the diameter of the protrusion 21 of the connecting plate 20. The protrusion 21 moves along the third guide groove 38. The third guide groove 38 is formed so that the protrusion 21 does not become caught therein.

The second restriction wall 36 includes a wall surface 36a (also referred to as a steep inclined surface), which has a steep gradient and defines the terminal end portion of the guide groove 34b, and a wall surface 36b (also referred to as a gradual inclined surface), which has gradual gradient and defines the terminal end portion of the third guide groove 38. In other words, the inclination angle of the wall surface 36b is smaller than the inclination angle of the wall surface 36a. Thus, when the protrusion 21 is guided from the third guide groove 38 toward the second restriction wall 36, the protrusion 21 easily moves from the third guide groove 38 to the guide groove 34b since the wall surface 36b has a gradual gradient. The first restriction wall 35 and the second restriction wall 36 have substantially the same height.

In the toothbrush sterilizer 1, the connecting plate 20 extending perpendicular to the front plate 12a connects the door 12 and the toothbrush holding tray 30. Thus, the toothbrush holding tray 30 operates in cooperation with the operation of the door 12. Specifically, the toothbrush holding tray 30 moves towards the front from the accommodation chamber S in cooperation with the opening operation of the door 12, and the toothbrush holding tray 30 moves towards the rear into the accommodation chamber S in cooperation with the closing operation of the door 12. The cooperative movement of the door 12 and the toothbrush holding tray 30 is achieved when the protrusion 21 moves along the first guide groove 34, the second guide groove 37, and the third guide groove 38, which are formed in the lower surface of the toothbrush holding tray 30.

In an embodiment, the door 12 is connected to the toothbrush holding tray 30 via a cam mechanism formed by the protrusion 21 of the connecting member 21 and the guide surface (34) of the toothbrush holding tray 30. The guide surface or bottom surface of the toothbrush holding tray 30 may be a face grooved cam, and the protrusion 21 of the connecting plate 20 may be a face cam pin.

The operation of the toothbrush holding tray 30 when opening the door 12 will now be described in detail.

Referring to Figs. 5 and 6(a), when the door 12 is in a closed state, the connecting plate 20 is accommodated in the accommodation chamber S overlapping the lower part of the toothbrush holding tray 30. In this state, the basal end of the connecting plate 20 is substantially flush with the front end of the toothbrush holding tray 30, and the distal end of the connecting plate 20 overhangs out of the rear open end of the first guide groove 34 in the toothbrush holding tray 30.

The user operates the door open switch 16 to open the door 12. As shown in Fig. 6(b), the protrusion 21 of the connecting plate 20 is guided by the guide groove 34a of the L-shaped first guide groove 34 and relatively moves towards the front along the guide groove 34a in cooperation with the opening operation of the door 12. During this movement, the protrusion 21 smoothly moves without being caught by the side surface of the guide groove 34a due to the relationship of the width of the guide groove 34a and the diameter of the protrusion 21 on the connecting plate 20.

As the user further continues the opening operation with the door 12, the protrusion 21 moves along a circle about the support shafts Q. The radius of the circle is the distance between the support shafts Q and the protrusion 21. As shown in Fig. 6(c), during the movement of the protrusion 21, the protrusion 21 contacts the side surface of the first guide groove 34 near the boundary between the guide groove 34a and the guide groove 34b (action point Q1). The movement of the protrusion 21 in the guide groove 34b is restricted by a certain extent due to the relationship of the width of the guide groove 34b and the diameter of the protrusion 21 of the connecting plate 20. Thus, contact of the protrusion 21 with the first guide groove 34 moves the toothbrush holding tray 30 towards the front along the rails R in cooperation with the opening operation of the door 12.

As the user further continues the opening operation with the door 12, the protrusion 21 moves along the guide groove 34b and reaches the first restriction wall 35 (see Figs. 6(d) and 6(e)). In this state, contact of the protrusion 21 with the first restriction wall 35 (wall surface 35a having steep gradient) restricts further movement of the protrusion 21, the connecting plate 20 is substantially parallel to the toothbrush holding tray 30, the connecting plate 20 substantially extends in the sideward direction Y2, and the door 12 stops at a position an which the open angle of the door 12 about the support shaft Q is about 90° (right angle). In this manner, the toothbrush holding tray 30 is drawn out and stopped at this frontmost position.

Thus, the toothbrush holding tray 30 does not need to be manually drawn out toward the front after opening the door 12. The toothbrush holding tray 30 is moved toward the front in cooperation with the opening of the door 12.

As shown in Fig. 6(d), in a state in which the toothbrush holding tray 30 is drawn out toward the front of the accommodation chamber S, the projection 20a of the connecting plate 20 contacts part of the bottom surface of the toothbrush holding tray 30 and supports the bottom surface of the toothbrush holding tray 30.

In the illustrated example, the protrusion 21 and the first restriction wall 35 serve as a restriction structure, and the projection 20a serves as a support for supporting the bottom surface of the toothbrush holding tray 30.

When the user further performs the opening operation with the door 12 from the state of Fig. 6(d), the connecting plate 20 flexibly deforms and the protrusion 21 climbs the wall surface 35a of the first restriction wall 35. Then, the protrusion 21 moves over the first restriction wall 35 and into the second guide groove 37. Afterward, the protrusion 21 moves along the second guide groove 37 and falls out from the open end of the second guide groove 37. This disconnects the connecting plate 20 from the toothbrush holding tray 30. After this disconnection, the pivoting of the door 12 is no longer restricted. Thus, the door 12 may open to an open angle of greater than 90° (obtuse angle) about the support shaft Q, for example, to a maximum open angle of 180°. In the illustrated example, the wall surface 35a of the first restriction wall 35 serves as a disconnecting structure.

The operation for returning the connecting plate 20 from the state of Fig. 7(a) to a state connected to the toothbrush holding tray 30 will now be discussed.

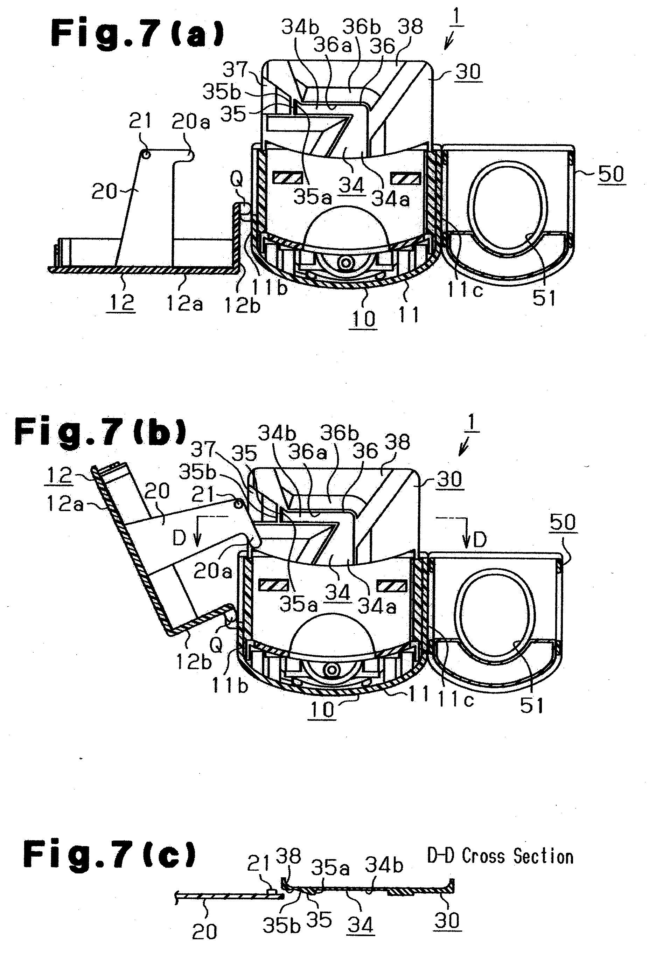

In the state of Fig. 7(a), the door 12 is open at an obtuse angle (open angle is 180°). The toothbrush holding tray 30 is drawn out to the frontmost position. When the user performs a closing operation with the door 12 from such state, the protrusion 21 of the connecting plate 20 enters the second guide groove 37 from the side of the toothbrush holding tray 30, as shown in Fig. 7(b). By further continuing the closing operation of the door 12, the protrusion 21 moves along the second guide groove 37 and climbs the wall surface 35b of the first restriction wall 35. Then, the protrusion 21 moves over the first restriction wall 35 and into the first guide groove 34. Since the wall surface 35b of the first restriction wall 35 has a gradual gradient (see Fig. 7(c)), the protrusion 21 smoothly moves over the first restriction wall 35. Movement of the protrusion 21 from the second guide groove 37 into the first guide groove 34 connects the door 12 and the toothbrush holding tray 30 with the connecting plate 20. In this manner, the protrusion 21 (connecting plate 20) is guided to a predetermined connection position and connected to the toothbrush holding tray 30 in such manner.

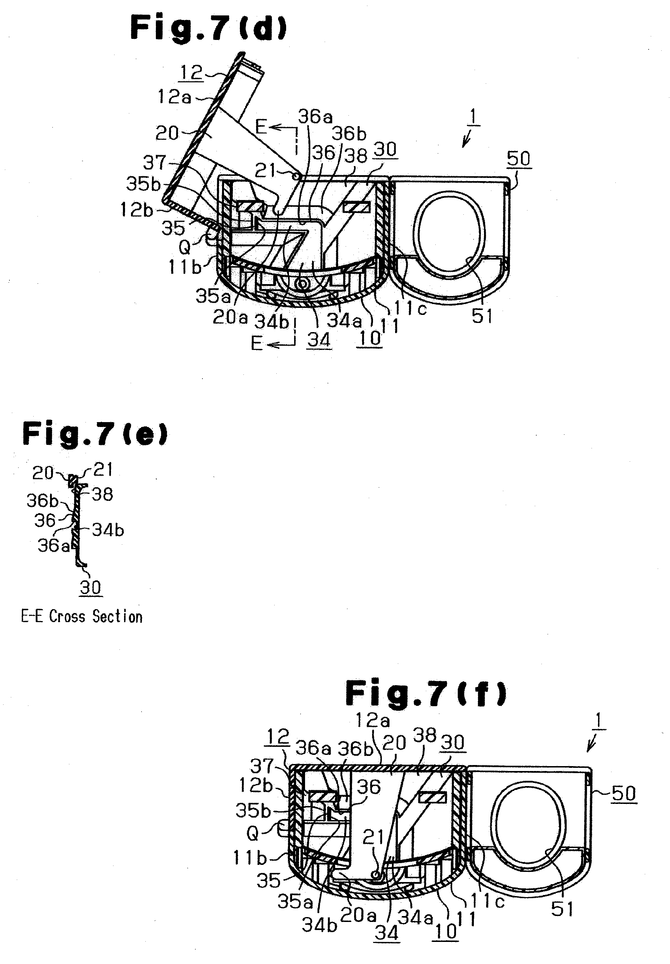

When the user further performs the closing operation with the door 12, the protrusion 21 is guided by the guide groove 34b to the guide groove 34a. Afterwards, operations are performed in an order reversed from the order of the operations performed when opening the door 12, as described above with reference to Figs. 6(b) to 6(d). That is, operations are performed in the order of Fig. 6(d) → Fig. 6(c) → Fig. 6(b). In cooperation with such closing operations of the door 12, the toothbrush holding tray 30, which is connected to the door 12 by the connecting plate 20, moves to the farther side of the accommodation chamber S while being guided by the rails R to be accommodated at the accommodation position shown in Fig. 7(f). The door 12 closes the opening K of the housing 10 when the toothbrush holding tray 30 is accommodated at the accommodation position.

In the toothbrush sterilizer 1 of this embodiment, the toothbrush holding tray 30 may be moved to and accommodated at the farther side of the housing 10 in cooperation with the closing operation of the door 12. Thus, there is no need for a separate manual operation to be performed to accommodate the toothbrush holding tray 30 when closing the door 12. In addition, the second guide groove 37 smoothly guides the connecting plate 20 to the predetermined connecting position of the toothbrush holding tray 30.

An example for inserting the connecting plate 20 from the front end of the lower surface of the toothbrush holding tray 30 to reconnect the door 12 and the toothbrush holding tray 30 will now be discussed.

As shown in Fig. 7(d), when the connecting plate 20 does not connect the door 12 and the toothbrush holding tray 30, and only the toothbrush holding tray 30 is accommodated at the accommodation position, the protrusion 21 of the connecting plate 20 is inserted into the third guide groove 38 from the front end of the lower surface of the toothbrush holding tray 30 to reconnect the door 12 and the toothbrush holding tray 30. To reconnect the door 12 and the toothbrush holding tray 30 with the connecting plate 20, the closing operation does not necessarily have to be performed with the door 12 in the state of Fig. 7(a). The door 12 and the toothbrush holding tray 30 may be reconnected even when the toothbrush holding tray 30 is located in the accommodation chamber S in which state the protrusion 21 cannot enter the second guide groove 37.

When the user performs the closing operation with the door 12 from the state of Fig. 7(d) (state in which the toothbrush holding tray 30 is completely accommodated in the accommodation chamber S), the protrusion 21 of the connecting plate 20 enters the third guide groove 38. As the user further performs the closing operation with the door 12, the protrusion 21 moves along the third guide groove 38 and onto the wall surface 36b of the second restriction wall 36. Then, the protrusion 21 moves over the second restriction wall 36 and into the first guide groove 34. As shown in Fig. 7(e), since the wall surface 36b of the second restriction wall 36 has a gradual gradient, the protrusion 21 smoothly moves over the second restriction wall 36. As the protrusion 21 moves from the third guide groove 38 to the first guide groove 34, the connecting plate 20 connects the door 12 and the toothbrush holding tray 30. In this manner, the protrusion 21 (connecting plate 20) is guided to a predetermined connecting position and connected to the toothbrush holding tray 30.

As the user further performs the closing operation with the door 12, the protrusion 21 is guided by the guide groove 34b to the guide groove 34a. Afterwards, operations are performed in an order reversed from the order of the operations performed when opening the door 12, as described above with reference to Figs. 6(b) and 6(d). That is, operations are performed in the order of Fig. 6(c) → Fig. 6(b). Such operations move the toothbrush holding tray 30, which is connected to the door 12 by the connecting plate 20, to the farther side of the accommodation chamber S while being guided by the rails R to be accommodated at the accommodation position shown in Fig. 7(f). The door 12 closes the opening K of the housing 10 when the toothbrush holding tray 30 is accommodated at the accommodation position.

In the toothbrush sterilizer 1 of this embodiment, the toothbrush holding tray 30 is moved in cooperation with the closing operation of the door 12 to the farther side of the housing 10 where it is accommodated. There is no need for a manual operation to be performed separately from the closing operation of the door 12 in order to accommodate the toothbrush holding tray 30 in the housing 10. In addition, the third guide groove 38 smoothly guides the connecting plate 20 to the predetermined connecting position of the toothbrush holding tray 30.

An operation for detaching the toothbrush holding tray 30 from the housing 10 will now be discussed.

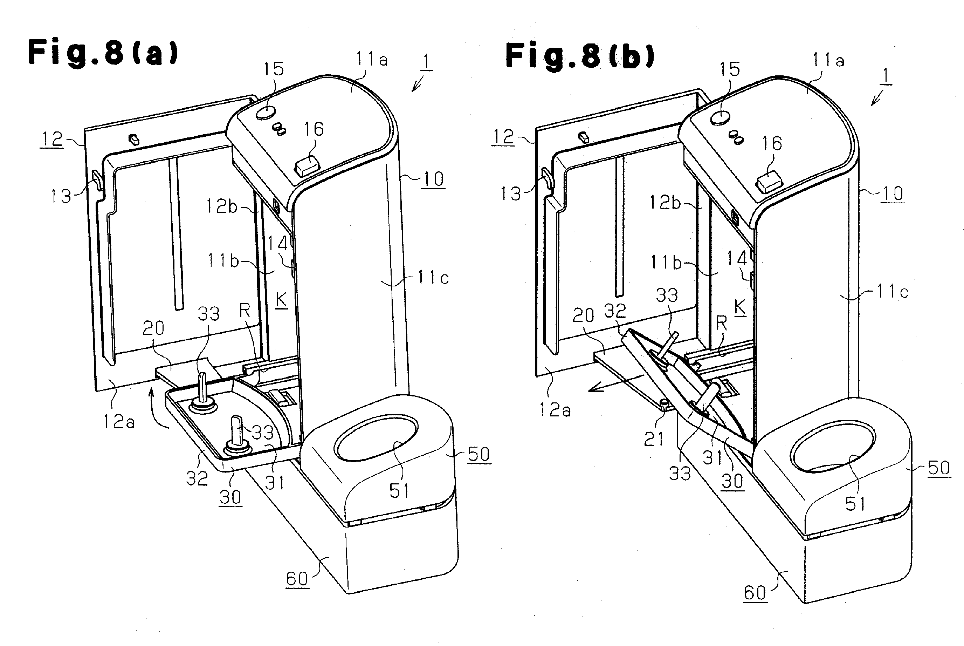

First, the user draws out the toothbrush holding tray 30 from the accommodation chamber S by opening the door 12. The front part of the toothbrush holding tray 30 is lifted in this state, as shown in Fig. 8(a), to pivot the toothbrush holding tray 30 about the protrusions Rp (extensions 32a) at the front end portions of the rails R. This easily disengages the extensions 32a of the toothbrush holding tray 30 from the protrusions Rp of the rails R, as shown in Fig. 8(b). The toothbrush holding tray 30 is detached by further pulling the toothbrush holding tray 30 from this state, as shown in Fig. 9.

The above-described embodiment has the advantages described below.
(1) The toothbrush holding tray 30 is formed to move in cooperation with the operation of the door 12. This eliminates the need for performing an operation that moves the toothbrush holding tray 30 in addition to the opening operation and closing operation of the door 12. The cooperation of the toothbrush holding tray 30 with the opening operation and closing operation of the door 12 improves convenience and hygienics.
(2) The toothbrush holding tray 30 is drawn out in cooperation with the opening operation of the door 12. Thus, the toothbrush H held by the toothbrush holding tray 30 is less likely to hit the wall of the toothbrush sterilizer 1. Further, when removing the toothbrush H from the toothbrush holding tray 30, the user is less likely to touch the bristle portion HS of the toothbrush H. This improves hygienics. The toothbrush H and the user's fingers are less likely to contact the UV lamp 17.
(3) The connecting plate 20, which connects the toothbrush holding tray 30 and the door 12, has a simple structure. Thus, a complicated structure for cooperative operation of the toothbrush holding tray 30 and the door 12 is unnecessary.
(4) The connecting plate 20 is connected to the lower surface of the toothbrush holding tray 30. Thus, the connecting plate 20 is less likely to be damaged by the UV lamp 17. The connecting plate 20 is also less likely to be stained by water dripping from the toothbrush H. Thus, the connecting plate 20 is easy to clean.
(5) The projection 20a of the connecting plate 20 supports the lower surface of the toothbrush holding tray 30. Thus, the toothbrush holding tray 30 is less likely to rattle. This stabilizes the movement of the toothbrush holding tray 30 and allows for a sophisticated appearance.
(6) The door 12 stops at a position where the open angle about the support shaft Q is substantially a right angle. This reduces the space occupied by the toothbrush sterilizer 1 during use.
(7) The second guide groove 37 opens at the of the toothbrush holding tray 30. This allows for disconnection of the door 12 and the toothbrush holding tray 30 from the side of the toothbrush holding tray 30 in which state restrictions imposed on the pivoting of the door 12 are eliminated. This allows for the user to freely use his or her dominant hand in any manner thereby improving convenience and hygienics.
(8) The second guide groove 37 for guiding the connecting plate 20 to the predetermined connecting position of the toothbrush holding tray 30 is formed on the lower surface of the toothbrush holding tray 30. When the connecting plate 20 and the toothbrush holding tray 30 are disconnected, the second guide groove 37 allows for easy reconnection of the connecting plate 20 and the toothbrush holding tray 30.
(9) The third guide groove 38 for guiding the connecting plate 20 to the predetermined connecting position of the toothbrush holding tray 30 is formed at a relatively frontward part of the toothbrush holding tray 30. Thus, even if the connecting plate 20 and the toothbrush holding tray 30 are disconnected and the toothbrush holding tray 30 is located in the accommodation chamber S at which the protrusion 21 cannot enter the second guide groove 37, by slightly increasing the force for closing the door 12, the protrusion 21 of the connecting plate 20 may be guided to the first guide groove 34 by the third guide groove 38 of the toothbrush holding tray 30. This easily connects the connecting plate 20 and the toothbrush holding tray 30.
(10) The first guide groove 34 is adjacent to the inclined wall surface 35a (steep inclined surface), and the second guide groove 37 is adjacent to the inclined wall surface 35b (gradual inclined surface). When opening the door 12, the protrusion 21 of the connecting plate 20 guided by the first guide groove 34 is hooks to the wall surface 35a. This holds the door 12 at an open angle of approximately 90°. The open angle of the door 12 is further widened when the user applies force to an extent that it unhooks the protrusion 21 from the inclined wall surface 35a. When closing the door 12 from the state in which the door 12 is greatly opened (state of approximately 90° or greater), the second guide groove 37 guides the protrusion 21 of the connecting plate 20 to the inclined wall surface 35b. This smoothly reconnects the connecting plate 20 and the toothbrush holding tray 30 as the door 12 closes.
(11) The third guide groove 38 is adjacent to the inclined wall surface 36b (gradual inclined surface). This smoothly reconnects the connecting plate 20 and the toothbrush holding tray 30.
(12) The toothbrush holding tray 30 is detachable from the housing 10. Thus, the toothbrush holding tray 30 is easy to clean.
(13) The door 12 pivots about the support shafts Q, which are arranged on the left panel 11b at substantially halfway positions of the toothbrush sterilizer 1 in the depthwise direction Y3. The location of the support shafts Q reduces the area occupied by the door 12 in the depthwise direction Y3 when opening the door 12. Thus, the toothbrush sterilizer 1 is convenient for used on a washstand.
(14) The toothbrush holding tray 30 is drawn out to the front in cooperation with the opening operation of the door 12. This eliminates the need for more upper space in the accommodation chamber S and allows for the toothbrush sterilizer 1 to be reduced in size.
(15) The widths of the first guide groove 34 (in particular, the guide groove 34a), the second guide groove 37, and the third guide groove 38 are much greater than the diameter of the protrusion 21. Thus, the protrusion 21 is less likely to be caught therein by side surfaces and therefore moves smoothly.

It should be apparent to those skilled in the art that the present invention may be embodied in many other specific forms without departing from the scope of the invention. Particularly, it should be understood that the present invention may be embodied in the following forms.

The number of toothbrushes H that can be held by the toothbrush holding tray 30 is not limited to two and may be one, three, or more.

In the above-described embodiment, the UV lamp 17 may stop the irradiation of ultraviolet rays when the door 12 opens and may irradiate ultraviolet rays when the door 12 closes.

In the above-described embodiment, the toothbrush sterilizer 1 includes the electric toothbrush holder 50, which is for charging an electric toothbrush. However, the electric toothbrush holder 50 may be omitted so that only sterilization of the toothbrush H is performed. In this case, the toothbrush sterilizer 1 may be reduced in size.

In the above-described embodiment, the left panel 11b may have substantially the same size as the right panel 11c, as shown in Fig. 10(a). This would increase the area occupied by the door 12 that overlaps with the toothbrush sterilizer 1 and thereby allow for the width of the toothbrush sterilizer 1 to be reduced. As shown in Fig. 10(b), the door 12 may be formed to pivot about a support shaft located at a rearmost position of the left panel 11b. This would increase the area occupied by the door 12 in the depthwise direction Y3 that overlaps the toothbrush sterilizer 1 and thereby allow for the dimension in the depthwise direction Y3 of the toothbrush sterilizer 1 to be reduced.

In the above-described embodiment, either one of the second guide groove 37 or the third guide groove 38 may be omitted.

In the above-described embodiment, the first restriction wall 35 may be formed by an elastic member. In such a case, when the protrusion 21 is guided from the guide groove 34b, the distal end of the first restriction wall 35 elastically deforms toward the second guide groove 37 and guides the protrusion 21 into the second guide groove 37. In the same manner, when the protrusion 21 is guided from the second guide groove 37, the distal end of the first restriction wall 35 elastically deforms toward the guide groove 34b and guides the protrusion 21 into the guide groove 34b.

In the above-described embodiment, the projection 20a of the connecting plate 20 may be omitted.

In the above-described embodiment, the connecting plate 20 does not have to be fixed to the door 12, and the connecting plate 20 may be detachable from the door 12.

In the above-described embodiment, the connecting member is not limited to the connecting plate 20. A connecting member such as spring can be used as long as it can connect the door 12 and the toothbrush holding tray 30.

In the above-described embodiment, the door 12 may open and close in the upward direction Y 1.

In the above-described embodiment, the toothbrush sterilizer 1 is not limited to sterilizing the toothbrush H portion of an electric toothbrush and may be used to sterilize a manual toothbrush.

In the above-described embodiment, the sterilization element is not limited to the UV lamp 17. Instead, a blue LED, an ozone generator, an ion generator, warm air generator, or the like may be used.

In the above-described embodiment, an insertion hole for receiving the toothbrush may be formed in the toothbrush holding tray 30.

The present examples and embodiments are to be considered as illustrative and not restrictive, and the invention is not to be limited to the details given herein, but may be modified within the scope and equivalence of the appended claims.

## Claims

1. A toothbrush sterilizer (1) for sterilizing a toothbrush (H), the toothbrush sterilizer (1) comprising:
a housing (10) having an opening (K);
a door (12) which opens and closes the opening (K);
a sterilization element (17); and
a toothbrush holding portion (30) which holds the toothbrush (H);
wherein the sterilization element (17) and the toothbrush holding portion (30) are accommodated in the housing (10), and the sterilization element (17) sterilizes the toothbrush (H); and
the toothbrush holding portion (30) moves out of the housing (10) through the opening (K) in cooperation with an opening operation of the door (12), and the toothbrush holding portion (30) moves into the housing (10) through the opening (K) in cooperation with a closing operation of the door (12).

2. The toothbrush sterilizer (1) according to claim 1, wherein the door (12) is connected to the housing (10) by a support shaft (Q) and pivotal about the support shaft (Q).

3. The toothbrush sterilizer (1) according to claim 2, wherein the toothbrush holding portion (30) is connected to the door (12) by a connecting member (20) and movable in cooperation with the opening operation and the closing operation of the door (12).

4. The toothbrush sterilizer (1) according to claim 3, wherein:
the toothbrush holding portion (30) includes a holder surface (31) on which the toothbrush (H) is held and a guide surface opposite the holder surface (31); and
the connecting member (20) connects the guide surface of the toothbrush holding portion (30) and the door (12).

5. The toothbrush sterilizer (1) according to claim 3, wherein:
the toothbrush holding portion (30) includes a holder surface (31) on which the toothbrush (H) is held and a guide surface opposite the holder surface (31); and
the connecting member (20) includes a support (20a) which supports the guide surface of the toothbrush holding portion (30).

6. The toothbrush sterilizer (1) according to claim 2, further comprising:
a restriction structure (21, 35) which restricts the opening operation of the door (12) at a position in which an open angle of the door (12) about the support shaft (Q) is substantially a right angle.

7. The toothbrush sterilizer (1) according to claim 2, further comprising:
a disconnecting structure (35a) which disconnects the door (12) and the toothbrush holding portion (30) that are connected by the connecting member (20);
wherein the disconnecting structure (35a) allows for the door (12) to open to an obtuse open angle about the support shaft (Q) when the disconnecting structure (35a) disconnects the door (12) and the toothbrush holding portion (30).

8. The toothbrush sterilizer (1) according to claim 7, wherein:
the toothbrush holding portion (30) includes a holder surface (31) on which the toothbrush (H) is held and a guide surface opposite the holder surface (31); and
the guide surface of the toothbrush holding portion (30) includes a guide (37) which guides the connecting member (20) to a predetermined connecting position on the toothbrush holding portion (30) during the closing operation of the door (12) from the obtuse open angle, with the guide having an open end in a side of the toothbrush holding portion (30).

9. The toothbrush sterilizer (1) according to claim 7, wherein:
the toothbrush holding portion (30) includes a holder surface (31) on which the toothbrush (H) is held and a guide surface opposite the holder surface (31); and
the guide surface of the toothbrush holding portion (30) includes a guide (38) which guides the connecting member (20) to a predetermined connecting position on the toothbrush holding portion (30) during the closing operation of the door (12) from the obtuse open angle, with the guide having an open end in a front end of the toothbrush holding portion (30).

10. The toothbrush sterilizer (1) according to claim 2, wherein the toothbrush holding portion (30) is detachable from the housing (10).

11. The toothbrush sterilizer (1) according to claim 3, wherein the connecting member (20) includes a basal end, which is fixed to the door (12), and a free end, which comes into contact with the guide surface of the toothbrush holding portion (30).

12. The toothbrush sterilizer (1) according to claim 11, wherein the connecting member (20) is a flexible plate extending substantially horizontally from the door (12).

13. The toothbrush sterilizer (1) according to claim 3, wherein the door (12) is connected to the toothbrush holding portion (30) via a cam mechanism (21, 34) formed by a portion (21) of the connecting member (20) and a portion (34) of the toothbrush holding portion (30).
